# EUROPEAN PATENT SPECIFICATION

(11) **EP 3 851 083 B1**
(45) Date of publication and mention of the grant of the patent: **19.06.2024**
(21) Application number: 19860352.4
(22) Date of filing: 28.08.2019
(51) Int. Cl.: A61F 13/00, A61F 13/36, A61F 13/44, A61L 15/28, A61L 15/42, D04B 21/00, D04B 21/12, D04B 21/16, A61L 15/22, A61L 15/54

(54) **MEDICAL GAUZE**
MEDIZINISCHER MULL
GAZE MÉDICALE

(30) Priority: 14.09.2018 JP 2018172687
(43) Date of publication of application: 21.07.2021
(73) Proprietor: Hakujuji Kabushiki Kaisha, Tokyo, 171-0033 (JP); Asahi Kasei Kabushiki Kaisha, Tokyo 100-0006 (JP)
(72) Inventor: SATO, Koichi, Tokyo 171-0033 (JP); OGURA, Hiroshi, Isesaki-Shi, Gunma 370-0124 (JP); ITO, Kenji, Fukui-Shi, Fukui 910-0843 (JP); HOSHIDA, Yusuke, Fukui-Shi, Fukui 910-0843 (JP); TAKEUCHI, Hisaharu, Osaka-Shi, Osaka 530-0005 (JP)
(74) Representative: Ablett, Graham Keith
(86) International application number: PCT/JP2019/033799
(87) International publication number: WO 2020/054434

(56) References cited:
- WO-A1-2006/115266
- WO-A1-2017/138653
- JP-A- 2005 177 034
- JP-A- 2012 097 376
- JP-A- 2013 002 028
- JP-A- 2013 227 689
- JP-U- 3 085 852
- JP-U- 3 130 538
- JP-U- 3 184 588
- JP-U- S5 218 596

## Description

### TECHNICAL FIELD

The present invention relates to medical gauze capable of absorbing blood or body fluids.

### BACKGROUND ART

Conventional medical gauze mainly used is cotton gauze prepared by plain weaving by using a large number of cotton yarns as warp yarn and weft yarn and crossing the warp yarn and weft yarn each other alternately.

In some cases, yarn waste is caused in the cotton gauze prepared by such plain weaving by the cotton yarn coming apart from a cutting plane. Further, as the cotton gauze is made of cotton yarn as a material prepared by spinning short fiber, part of the fiber forming the cotton yarn can drop from the gauze during use to cause lint.

The lint is dropping fiber such as short fiber trash dropping from the gauze.

The medical gauze of this type is used temporarily in a body for absorption of body fluids or blood or for blood stanching during an operation, for example. Hence, the occurrence of yarn waste or lint can be perceived as a problem, particularly by medical workers involved in surgery. A report has been issued so far relating to a medical accident caused by entry of yarn waste or lint into a blood-vascular system.

In this regard, medical gauze configured using a woven fabric containing substantially no foreign matters has been known, as disclosed in PTL 1.

While the medical gauze in PTL 1 is formed by the conventional method of plain weaving warp yarn and weft yarn, regenerated fiber-based yarn is used as at least one of the warp yarn and the weft yarn.

Regenerated fiber is regeneration of natural fiber such as wood pulp or cotton as fiber prepared by dissolving the wood pulp or cotton and injecting the dissolved solution from a nozzle into a coagulating solution.

Fat or foreign matters contained in the natural fiber are also dissolved during dissolving of the natural fiber. Thus, the fat or foreign matters attached to the natural fiber are absent in the regenerated fiber.

For this reason, using the regenerated fiber as both warp yarn and weft yarn achieves considerable reduction in lint, compared to medical gauze in which normal cotton yarn is used as warp yarn and weft yarn. Using the regenerated fiber as one of warp yarn and weft yarn and using cotton yarn as the other is expected to reduce the amount of lint by about half.

According to a disclosure in PTL 2, to improve lint-free performance by reducing foreign matters, a cellulose fiber nonwoven fabric more excellent in lint-free performance than cotton gauze is provided at one side or both sides of cotton gauze to form a composite of the cellulose fiber nonwoven fabric and the cotton gauze integrated with each other.

### CITATION LISTPatent Literatures

PTL 1: Japanese Laid-open Utility Model Registration No. 3184588; PTL 2: Japanese Laid-open Patent Publication No. 2002-238943; PTL 3: WO2006/115266.

### SUMMARY OF INVENTION

### Technical Problem

The gauze in PTL 1 is gauze as a plain woven fabric using regenerated fiber yarn as both or one of warp yarn and weft yarn. The regenerated fiber yarn has a smoother surface and has a considerably lower frictional resistance than cotton yarn. Hence, plain weaving using the regenerated fiber yarn as both the warp yarn and the weft yarn makes yarns crossing each other easy to slip. This makes the warp yarn and the weft yarn easy to move relative to each other if the warp yarn and the weft yarn are in a density (the number of yarns/cm) substantially equal to that in the conventional cotton gauze, unfortunately causing a mesh structure in which mesh distortion, namely, slippage is likely to occur.

Using regenerated fiber yarn cotton yarn as only one of the warp yarn and the weft yarn for suppressing slippage certainly results in the occurrence of more lint than using regenerated fiber yarn as both the warp yarn and the weft yarn.

The gauze disclosed in PTL 2 achieves improvement of lint-free performance compared to the conventional cotton gauze. However, using a nonwoven fabric made of short fiber of cellulose fiber in an upper layer or in both the upper layer and a lower layer causes a probability of dropping of such short fiber.

Using a nonwoven fabric made of cellulose continuous long fiber in the upper layer or in both the upper and lower layers improves lint-free performance, compared to using a short-fiber nonwoven fabric. However, it becomes likely that fiber in the cotton gauze used in an intermediate layer or in a cellulose short fiber layer will be exposed to the upper and lower layers or the short fiber will drop during cutting of the gauze.

Thus, medical gauze of a configuration allowing improvement of lint-free performance more effectively has been desired.

The present invention has been made in view of the foregoing points, and is intended to provide medical gauze with favorable lint-free performance.

### Solution to Problem

Medical gauze according to claim 1 includes a warp knitted fabric having a warp direction and a weft direction and composed of loops continued in the warp direction, and x-ray contrast yarn interlaced in the warp knitted fabric. The warp knitted fabric is made of 100% long fiber containing 70% by weight or more of regenerated cellulose-based long fiber. At least one chemical fiber is interwined with the x-ray contrast yarn in the warp direction and similar chemical fiber is arranged in the warp direction in the vicinity of the at least one chmical fiber.

Medical gauze according to claim 2 is such that, in the medical gauze according to claim 1, the warp knitted fabric is configured in a marquisette construction.

Medical gauze according to claim 3 is such that, in the medical gauze according to claim 1 or 2, the regenerated cellulose-based long fiber is cuprammonium rayon.

Medical gauze according to claim 4 is such that, in the medical gauze according to any one of claims 1 to 3, the medical gauze has a width of equal to or greater than 5 mm and equal to or less than 600 mm, and a weight of equal to or greater.

According to the invention, the x-ray contrast yarn is interlaced in the warp knitted fabric.

Further, at least one chemical fiber is intertwined with the x-ray contrast yarn in the warp direction, and similar chemical fiber is arranged in the warp direction in the vicinity of the chemical fiber.

Medical gauze according to claim 5 is such that the total number of the chemical fibers is between four and eight.

Medical gauze according to claim 6 is such that the chemical fiber is polyethylene terephthalate fiber.

### Advantageous Effects of Invention

According to the present invention, as the warp knitted fabric is composed of the loops continued in the warp direction and made of 100% long fiber containing 70% by weight or more of the regenerated cellulose-based long fiber, the occurrence of lint can be reduced to allow improvement of lint-free performance.

### BRIEF DESCRIPTION OF DRAWINGS

[Fig. 1]

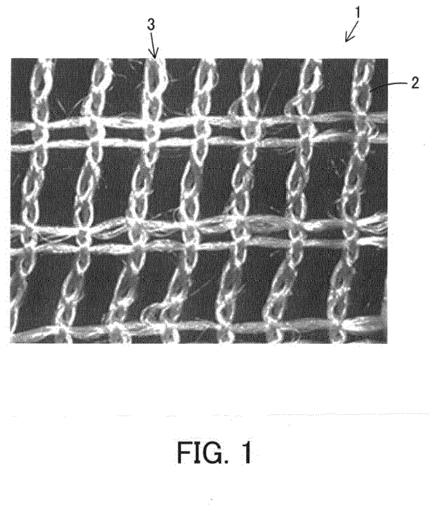

   Fig. 1 is a photograph showing the construction of a warp knitted fabric of medical gauze;
[Fig. 2]

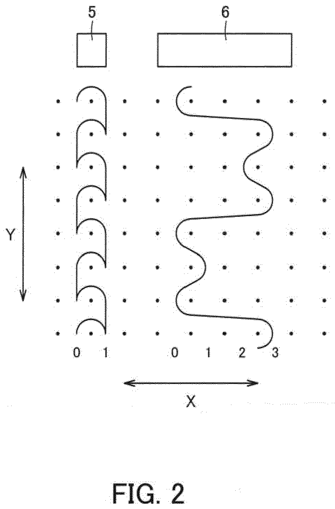

   Fig. 2 is an explanatory view schematically showing a marquisette construction forming the warp knitted fabric of the medical gauze;
[Fig. 3]

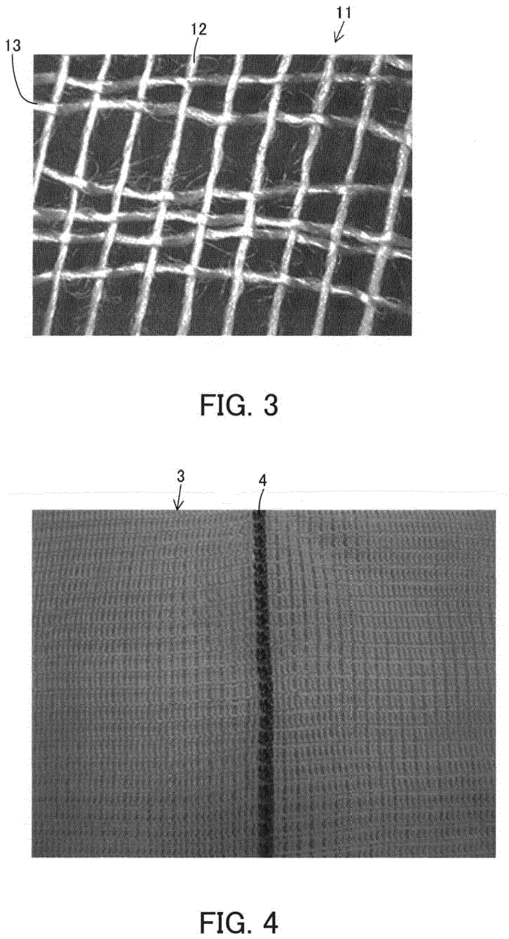

   Fig. 3 is a photograph showing the construction of conventional medical gauze;
[Fig. 4]
   Fig. 4 is a photograph showing the configuration of a modification of the medical gauze;
[Fig. 5]

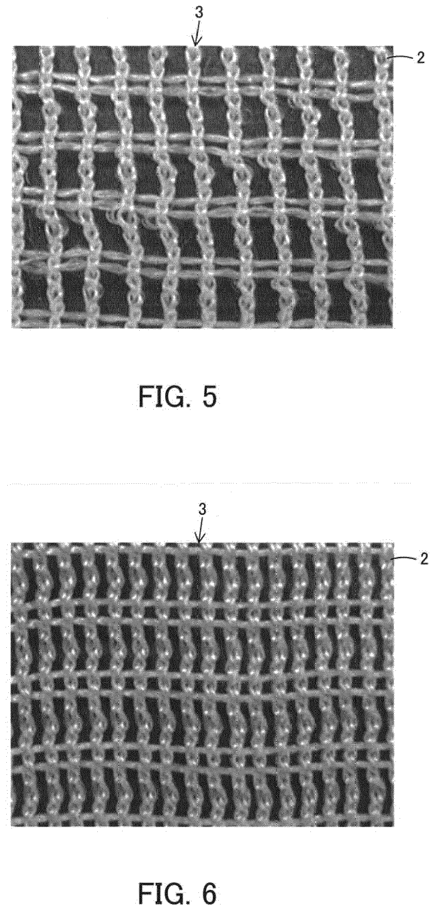

   Fig. 5 is a photograph showing the construction of a warp knitted fabric according to Example 1;
[Fig. 6]
   Fig. 6 is a photograph showing the construction of a warp knitted fabric according to Example 6;
[Fig. 7]

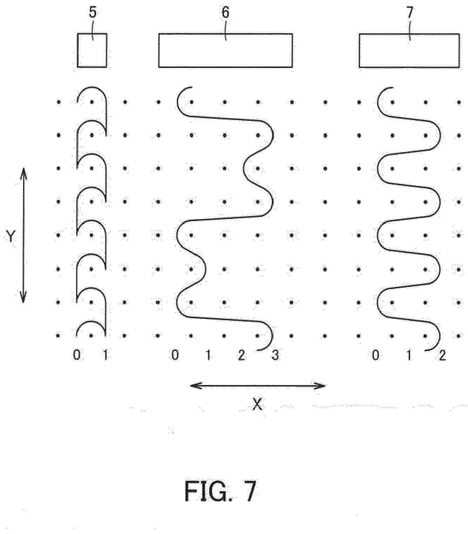

   Fig. 7 is an explanatory view schematically showing a marquisette construction forming a warp knitted fabric according to Example 5;
[Fig. 8]

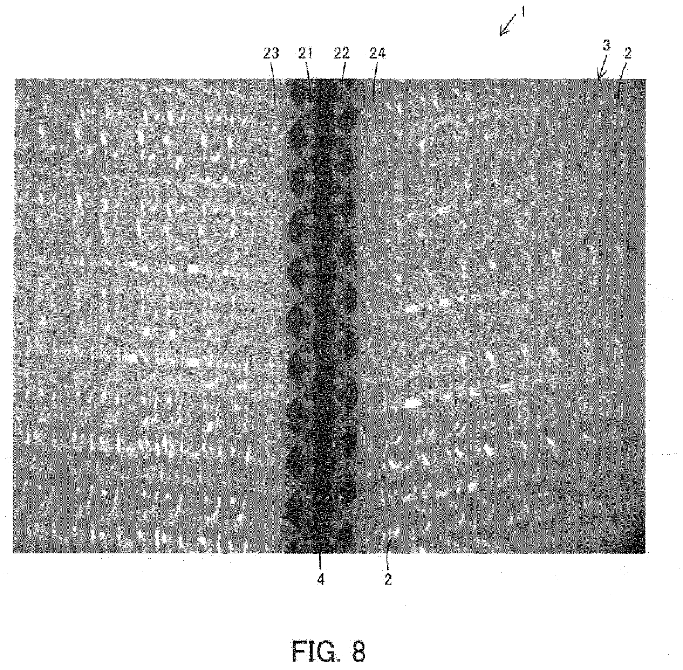

   Fig. 8 is a photograph showing the configuration of medical gauze according to an embodiment of the present invention;
[Fig. 9]

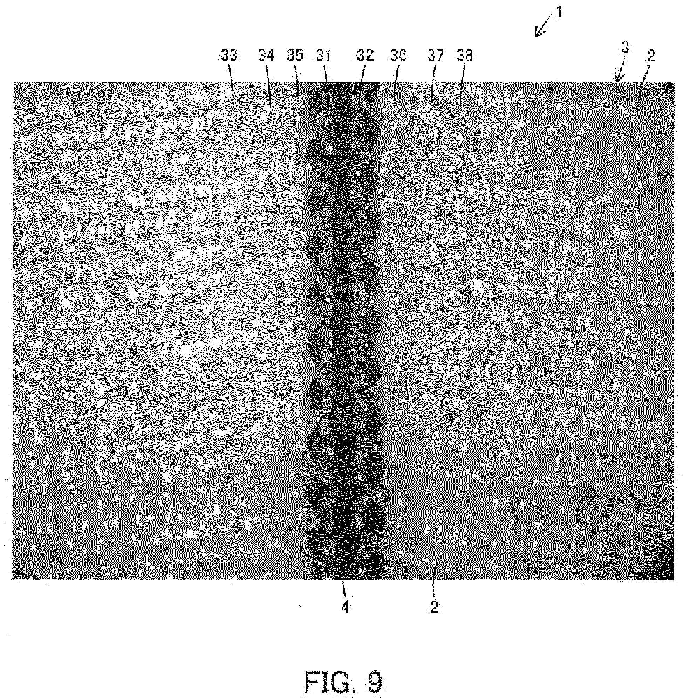

   Fig. 9 is a photograph showing the configuration of medical gauze according to another embodiment of the present invention;
[Fig. 10]
   Fig. 10 shows the structure of x-ray contrast yarn; [Fig. 11]
Fig. 11 is a photograph showing a specimen of a strength test;
[Fig. 12]

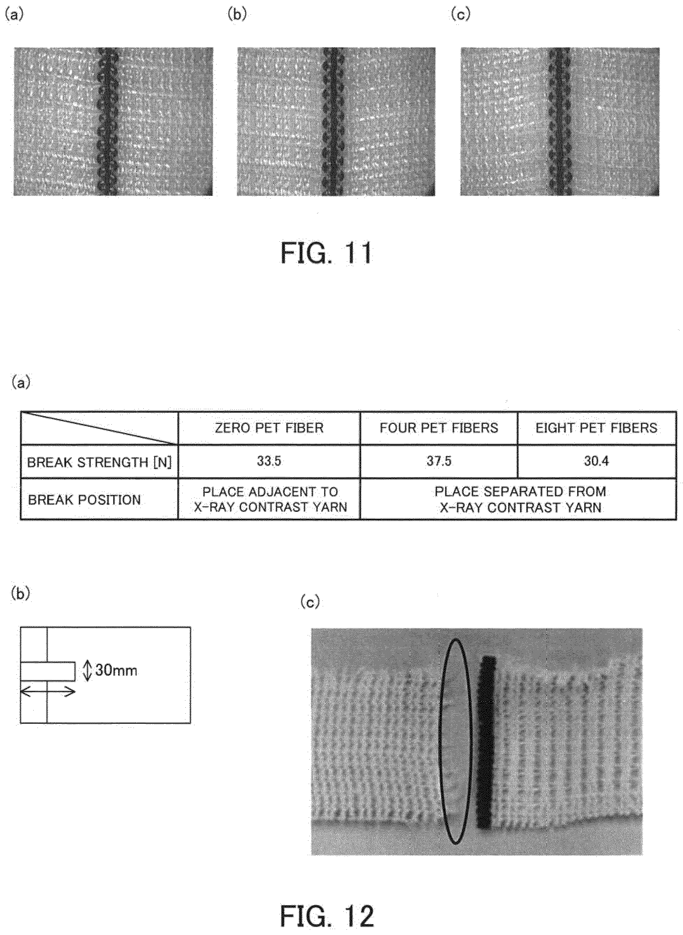

   Fig. 12 includes a table and the like showing test result of the strength test;
[Fig. 13]

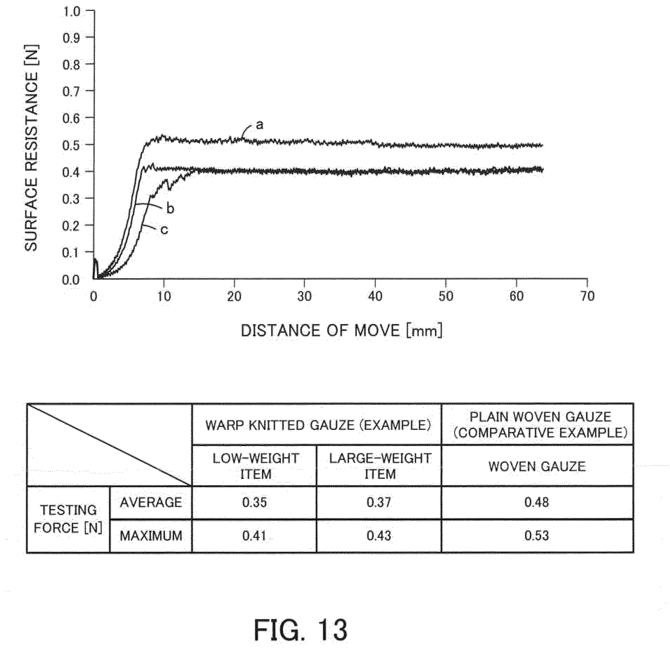

   Fig. 13 includes a graph and the like showing test result of a surface resistance check test (a PP plate with a smooth surface);
[Fig. 14]

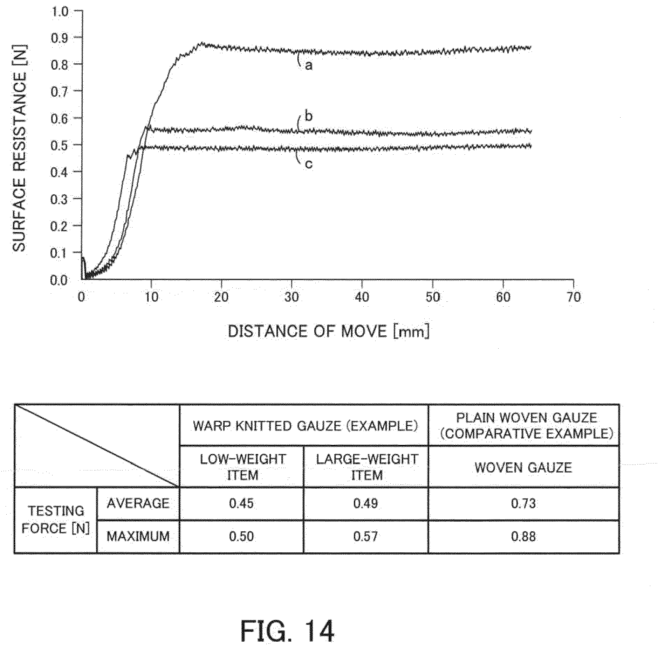

   Fig. 14 includes a graph and the like showing test result of the surface resistance check test (a PP plate with an uneven surface);
[Fig. 15]

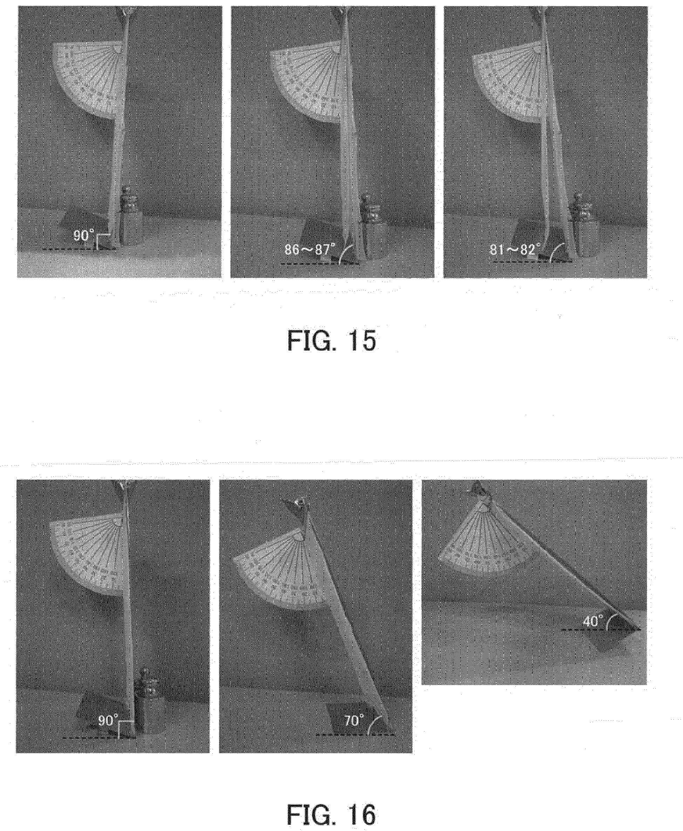

   Fig. 15 is a photograph showing test result of a fabric detachability check test (two warp knitted gauze pieces);
[Fig. 16]
   Fig. 16 is a photograph showing test result of the fabric detachability check test (two plain woven cotton gauze pieces); and
[Fig. 17]

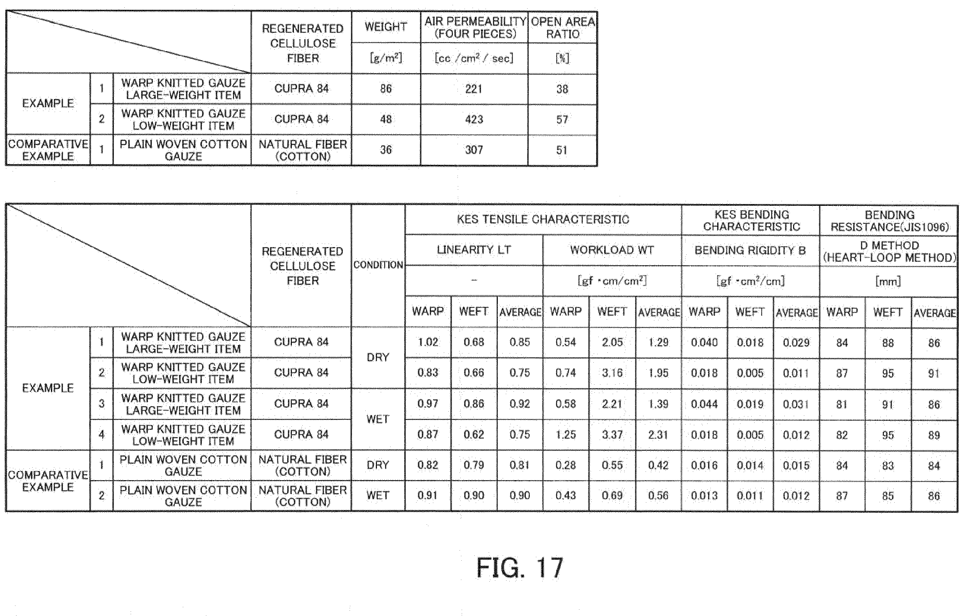

   Fig. 17 is a table showing test result of a performance check test.

### DETAILED DESCRIPTION

The configuration of the present invention will be described below in detail by referring to at least Figures 8 and 9 of the drawings. Other examples and configurations are maintained herein for completeness.

Referring to Fig. 1, 1 shows medical gauze to be used for absorption of body fluids or blood, blood stanching, covering of a wound surface, etc. during implementation of medical practice such as an operation, for example.

The medical gauze 1 includes a warp knitted fabric 3 of a warp knitted construction composed of loops 2 continued in a warp direction formed by a warp knitting method.

The warp knitted fabric 3 is configured in a marquisette construction. The marquisette construction is prepared by forming chain stitches (loops) using a front reed, making a combination of inlaid knitting with two or more needles and one-needle interval inlaid knitting using one or two back reeds for connecting the chain stitches formed using the front reed, and making such combinations along several courses. That is, the marquisette construction is prepared by knitting yarn in the warp direction into a chain, connecting adjacent chain stitches to each other with inlaid yarn extending further in the weft direction, and forming the warp knitted fabric 3 as a whole into a square mesh-like shape.

The marquisette construction will be described in more detail. As shown in Fig. 2, yarn from a first guide bar 5 forms a construction (chain construction) interlaced on the same needle with loops formed in the warp direction and with no connection between adjacent yarns. In Figs. 2 and 7, an X direction corresponds to a wale direction and a Y direction corresponds to a course direction.

Yarn from a second guide bar 6 forms a construction (inlaid construction) by repeating movements of moving for three needles in the weft direction without being used for interlacing, then moving for one needle in the opposite direction, then moving further for one needle in the opposite direction, and thereafter moving for three needles in the opposite direction.

Yarn is aligned in all guides in both of the first guide bar 5 and the second guide bar 6.

Using only the first guide bar 5 does not provide connection between yarns adjacent to each other, so that a resultant construction does not fulfill a function as a fabric. Using only the second guide bar 6 does not achieve interlacing, so that a resultant construction does not fulfill a function as a fabric. However, combining these two constructions provides connection in the weft direction and achieves interlacing to fix an inlaid part, thereby fulfilling formation as a fabric.

For reference, with the first guide bar 5 defined as GB1 and the second guide bar 6 as GB2, the marquisette construction is expressed in a numerical form as follows: GB1: 01-10 and GB2: 33-00-11-00.

The warp knitted fabric 3 is made of 100% long fiber containing 70% by weight or more of regenerated cellulose-based long fiber.

The long fiber is one continuous long yarn (filament yarn) with no cut. The warp knitted fabric 3 is formed by a warp knitting method using such long fiber.

Regarding the warp knitted fabric 3, the percentage by weight of regenerated cellulose-based long fiber of being less than 70% by weight reduces fluid absorbing property, causing a probability of failing to absorb body fluids or blood sufficiently in medical use or a probability of softness reduction during fluid absorption. In this regard, the percentage of the regenerated cellulose-based long fiber in the long fiber is set at equal to or greater than 70% by weight.

To absorb blood or body fluids efficiently, the conventional medical gauze is generally required to be made of a material with high water absorbing property. However, a material for a surgical operation and the like is not always required to have high water absorbing property. In such cases, for use for blood stanching purpose, a material to absorb a small quantity of body fluid such as blood may be required. For this reason, in addition to the water-absorbing regenerated cellulose-based long fiber, synthetic fiber with no water absorbing property such as nylon, polyester, or polypropylene may also be used to control fluid absorbing property appropriately according to an expected purpose of use.

As the regenerated cellulose-based long fiber is one continuous long fiber with no cut, it does not cause short fiber trash. By contrast, as cotton yarn is spun yarn prepared by tying and twisting short fibers together, it is likely to cause fiber trash.

The regenerated cellulose-based long fiber is highly-pure chemical fiber prepared by dissolving a natural product such as pulp or cotton linter in a chemical agent, extracting plant cellulose chemically, and regenerating the extracted plant cellulose into fiber. Conventional cotton gauze requires scouring process by means of degreasing process and bleaching process after weaving. By contrast, as the warp knitted fabric 3 is made of the regenerated cellulose-based long fiber having high cellulose purity and high water absorbing property, it does not require degreasing process intended for washing away impurity and applying water absorbing property.

The degreasing process is performed by boiling gauze in a high-concentration alkaline liquid under high temperature and high pressure. Thus spun cotton yarn (short fiber) may be loosen and this can inadvertently cause fiber trash on the verge of coming off during cutting of the gauze to drop during use of the gauze.

More specifically, the regenerated cellulose-based long fiber is preferably viscose rayon (rayon), cuprammonium rayon (cupra), or solvent spun fiber (Lyocell), for example, having more favorable absorbing property than other types of cellulose fiber, fulfilling excellent softness during fluid absorption, and containing a small amount of mixed impurity other than cellulose.

Also, the total size of the regenerated cellulose-based long fiber to be used can be selected appropriately from a range of equal to or greater than 30 dtex and equal to or less than 200 dtex. For manufacturing the medical gauze 1 with the warp knitted fabric 3 having an appearance and mechanical strength quite similar to those of conventional cotton gauze (a weight of 36 g/m²) , a preferable range is equal to or greater than 50 dtex and equal to or less than 100 dtex.

Further, the single yarn size (filament size) of the regenerated cellulose-based long fiber is not particularly limited and in a range from 0.5 to 5 dtex. From a viewpoint of knitting performance and grade during implementation of warp knitting and the softness of warp knitted gauze, a preferable range is from 1 to 3 dtex.

In addition, the regenerated cellulose-based long fiber is available, either it is untwisted or twisted. To use the twisted regenerated cellulose-based long fiber, the number of twisted yarns is selected appropriately in response to the total size of fiber to be used.

Further, the regenerated cellulose-based long fiber may be bulky textured yarn. Examples of available yarn include false-twisted textured yarn, force textured yarn, textured yarn with loop pile prepared by intertwining long-fiber single yarns with each other using compressed air, and chenille yarn.

These types of bulky textured yarn to be used can also be yarn having a composite structure of the regenerated cellulose-based long fiber and synthetic resin such as polyester or nylon, for example.

The medical gauze 1 configured using the warp knitted fabric 3 described above is required to be sterilized for use in a case such as an operation. Thus, the medical gauze 1 is preferably given guaranteed sterilization by formation into the shape of the medical gauze 1 and then implementation of scouring process and sterilizing process.

The scouring process is preferably performed by scouring the warp knitted fabric 3 at about 70°C using a bleaching agent such as hydrogen peroxide water. Implementation of this scouring process makes it possible to reduce the number of living bacteria in the warp knitted fabric 3, compared to that at the time of finish of the warp knitting.

It is noted that the bleaching process using the bleaching agent is not an absolute necessity in the scouring process. For the purpose of smoothing and softening a fabric only, the scouring process to be applied may also be performed only through dipping into warm water.

The sterilizing process after the scouring process is preferably performed through sterilization using ethylene oxide gas (EOG sterilization) or through sterilization using high-pressure steam(AC sterilization). A product to be formed is preferably guaranteed sufficiently in terms of sterilization by the implementation of the sterilizing process.

Also, in consideration of use in a surgical operation, the medical gauze 1 preferably has a width of equal to or greater than 5 mm and equal to or less than 600 mm.

Further, the weight of the warp knitted fabric 3 of less than 28 g per square meter causes a probability of failing to ensure water absorbing property sufficiently in the medical gauze 1. On the other hand, conventional plain woven gauze normally has a weight of 36 g per square meter. In some causes, eight pieces of such gauze are stacked and sewed together to manufacture thick gauze having a weight of 288 g per square meter. Hence, use of the medical gauze 1 having a similar weight is also expected. For this reason, in the medical gauze 1, the warp knitted fabric 3 preferably has a weight of equal to or greater than 28 g and equal to or less than 290 g per square meter.

The action and effect of the foregoing will be described next.

In the foregoing medical gauze 1, the warp knitted fabric 3 is composed of the loops 2 continued in the warp direction and is formed only of long fiber that is continuous yarn with no cut. This makes fiber unlikely to drop not only in use in a normal state but also in use in a cut state, making it possible to improve lint-free performance effectively.

Also, the long fiber contains 70% by weight or more of the regenerated cellulose-based long fiber. This makes it possible to ensure water absorbing property and softness for medical use as well as to improve lint-free performance.

Here, comparison is made between the medical gauze 1 shown in Fig. 1 and gauze 11 shown in Fig. 3 as Comparative Example prepared by plain weaving warp yarn 12 and weft yarn 13 using regenerated cellulose-based long fiber. While the medical gauze 1 has a regulated knitted construction, the woven construction of the gauze according to Comparative Example formed by a plain weaving method is not regulated. More specifically, the regenerated cellulose-based long fiber has a smooth surface so the frictional resistance thereof is low. Hence, as a result of implementation of the plain weaving method of simply crossing the warp yarn and the weft yarn, the warp yarn and the weft yarn are caused to move and misaligned without being fixed each other. In the medical gauze 1 formed by the warp knitting method, however, yarn is interlaced in a complicated fashion. This makes it possible to retain a regulated knitted construction even by the use of the regenerated cellulose-based long fiber, as well as to reduce a probability of dropping of fiber.

The warp knitted fabric 3 is configured in the marquisette construction. By doing so, a stable shape is easily retained using a chain construction continued in the warp direction and inlaid yarn stretching in the weft direction.

The marquisette construction is also unlikely to cause defective stitches, even if it is thin or low-weight. This allows improvement of dimensional stability.

Formation using the warp knitting method further allows interlacing in a thickness direction while such interlacing is infeasible by the plain weaving method. This allows the weight of gauze to be changed freely by increasing the fiber diameter of yarn or increasing a swing range of an inlaid construction, thereby facilitating formation into a configuration responsive to a purpose of use.

Using viscose rayon, cuprammonium rayon, or solvent spun fiber, particularly using cuprammonium rayon as the regenerated cellulose-based long fiber makes it possible to ensure proper water absorbing property and improve softness, thereby achieving improvement of the feel of use and usability.

The medical gauze 1 is made available for use in a surgical operation by setting the width dimension of the medical gauze 1 at equal to or greater than 5 mm and equal to or less than 600 mm, and setting the weight thereof at equal to or greater than 28 g and equal to or less than 290 g per square meter.

It is noted that the gauze is not limited to the foregoing configuration described above but can be changeable, if appropriate, as long as the configuration includes a warp knitted fabric composed of loops continued in the warp direction, the warp knitted fabric is made of 100% long fiber containing 70% by weight or more of regenerated cellulose-based long fiber.

Namely, as shown in Fig. 4, for example, a configuration including x-ray contrast yarn 4 interlaced in the warp knitted fabric 3 is applicable.

During warp knitting, the x-ray contrast yarn 4 is preferably aligned between the regenerated cellulose-based long fibers in the warp knitted fabric 3.

The x-ray contrast yarn 4 is prepared by adding barium sulfate, for example, having x-ray contrast effect into plastic resin such as polyvinyl chloride, polypropylene, polystyrene, or silicon, or regenerated cellulose-based long fiber such as cuprammonium rayon, for example.

Also, the x-ray contrast yarn 4 to be used may either be monofilament yarn formed of a single filament, or a multifilament formed by mixing an x-ray contrast agent into fibrous resin.

The x-ray contrast yarn 4 may further be composite yarn prepared by twisting the x-ray contrast yarn 4 with other synthetic resin or regenerated cellulose fiber with no x-ray contrast property, or by covering the x-ray contrast yarn 4 with such resin or fiber.

After being used in a surgical operation and the like, the medical gauze 1 is removed from a body after the operation. As the medical gauze 1 is frequently stained with blood during the operation, it becomes difficult to distinguish the medical gauze 1 from an organ, and the like. This causes a risk of proceeding to suture and finishing the operation while the medical gauze 1 is left in the body.

In this regard, arranging the x-ray contrast yarn 4 in the warp knitted fabric 3 makes it possible to check the retention of the medical gauze 1 easily in an x-ray photograph taken after the operation.

It is noted that, while medical gauze with x-ray contrast yarn has conventionally been known, the x-ray contrast yarn 4 is generally prepared by weaving yarn thicker than cotton yarn (generally, 40-cotton yarn) in order to facilitate check to see the presence or absence of the retention in the body.

Such thick x-ray contrast yarn causes a probability that weaving performance will be deteriorated or the x-ray contrast yarn will drop easily. However, in the medical gauze 1 according to the present invention, interlacing the x-ray contrast yarn into the warp knitted fabric 3 can prevent deterioration of knitting performance or dropping of the x-ray contrast yarn 4.

The x-ray contrast yarn 4 can also be attached by welding as described later. However, forming the x-ray contrast yarn 4 as a part of the warp knitted fabric 3 achieves a higher level of x-ray contrast in an x-ray photograph than the attachment by welding.

If forming the x-ray contrast yarn 4 as a part of the warp knitted fabric 3 is impossible, the x-ray contrast yarn 4 can be attached easily by welding.

The x-ray contrast yarn 4 can be attached by welding by melting plastic resin (polyvinyl chloride, polypropylene, polystyrene, or silicon, for example) forming the x-ray contrast yarn 4 using a heat or ultrasonic welding machine, and pressing the resin under pressure against the warp knitted fabric 3.

However, a medical gauze 1 of the claimed configuration is shown in Fig. 8 or a configuration shown in Fig. 9, for example.

In the configuration shown in Fig. 8, at least one chemical fiber, specifically, two chemical fibers (intertwined fibers) 21 and 22, for example, are intertwined with one x-ray contrast yarn (two needle swing) 4 thicker than the chemical fibers 21 and 22 in the warp direction. Further, two chemical fibers 23 and 24 similar to the chemical fibers 21 and 22 are arranged in the warp direction in the vicinity of the chemical fibers 21 and 22 (at neighboring positions on the right and left sides of the x-ray contrast yarn 4) without being intertwined with the x-ray contrast yarn 4. Therefore, the total number of the chemical fibers 21 to 24 used as replacement for the long fiber is four. All the chemical fibers 21 to 24 are made of the same long fiber that is polyethylene terephthalate fiber (PET fiber), for example, having higher fiber strength and more favorable heat resistance than the long fiber before the replacement (regenerated cellulose-based long fiber such as cupra) . It is noted that, like the long fiber before the replacement, each of the chemical fibers 21 to 24 has a chain construction with the loops 2 continued in the warp direction.

In the configuration shown in Fig. 9, the total number of chemical fibers is eight, which is larger than that of the foregoing configuration shown in Fig. 8. For example, two chemical fibers (intertwined fibers) 31 and 32 are intertwined with one x-ray contrast yarn (two needle swing) 4 thicker than the chemical fibers 31 and 32, and six chemical fibers 33 to 38 similar to the chemical fibers 31 and 32 are arranged in the warp direction in the vicinity of the chemical fibers 31 and 32 without being intertwined with the x-ray contrast yarn 4. Like the foregoing chemical fibers 21 to 24 shown in Fig. 8, for example, all the chemical fibers 31 to 38 are made of the same PET long fiber.

The medical gauze 1 having the configuration described above shown in Fig. 8 or 9 achieves increased strength in an area around the x-ray contrast yarn 4 in addition to achieving the foregoing effect such as improvement of lint-free performance. Further, even if the medical gauze 1 is broken by pulling, this break occurs at a boundary area between the long fiber and the PET fiber (an area separated from the x-ray contrast yarn 4), thereby preventing dropping of the x-ray contrast yarn 4.

It is noted that the number of the foregoing chemical fibers is not always required to be an even number (four, six, or eight, for example), and these chemical fibers are not always required to be arranged bilaterally symmetrically. In consideration of right and left balance, however, the same number of chemical fibers are preferably arranged on the right and left sides of the x-ray contrast yarn 4.

In addition, yarn breakage can be prevented by making change to chemical fiber having higher fiber strength than long fiber. The chemical fiber to be used as replacement for the long fiber is preferably PET fiber having high heat resistance in consideration of the scouring process. It is noted that, not using PET fiber is not preferable, as it causes a risk of dropping of the x-ray contrast yarn 4 from the warp knitted yarn 3 if the long fiber is cut in a place adjacent to the x-ray contrast yarn 4 to break the warp knitted fabric 3.

On the other hand, as shown in Fig. 7, the x-ray contrast yarn 4 is intertwined with two long fibers. Thus, intertwining at least two chemical fibers (PET fibers, for example) as replacement for the long fibers with the x-ray contrast yarn 4 allows the chemical fibers to function to reduce the occurrence of break of the long fiber during pulling of the warp knitted fabric 3. By doing so, strength at the intertwining area can be reinforced. However, using only the two chemical fibers fails to remove stress on the long fiber completely to be applied in the area where the chemical fibers and the long fiber are intertwined with each other during pulling of the warp knitted fabric 3. In some cases, this cannot be considered to be sufficient for the reinforcement. For this reason, the number of the chemical fibers is preferably equal to or greater than four. However, the chemical fibers of a number more than necessary inadvertently harden an area around the x-ray contrast yarn 4. Thus, an upper limit number is preferably eight.

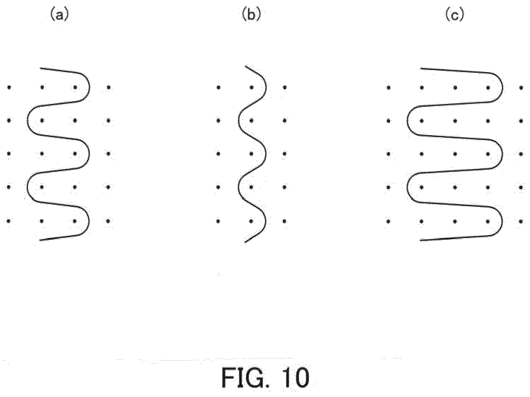

For the reason given above, the total number of chemical fibers is preferably between four and eight. However, the number of chemical fibers to be intertwined with x-ray contrast yarn may be at least one or more. In consideration of the point that the minimum number of chemical fibers settable at a knitting machine (marquisette construction) is two, the minimum number of chemical fibers to be intertwined with the x-ray contrast yarn is preferably two. It is noted that, in addition to being the two needle swing yarn shown in Fig. 10 (a), the x-ray contrast yarn may be one needle swing yarn shown in Fig. 10(b) or three needle swing yarn shown in Fig. 10(c).

### Examples

Examples and Comparative Examples will be described below.

Medical gauze was formed into a configuration shown in Table 1, and then performance evaluation was conducted.

According to the JIS L 1096A method, the weight of the gauze was calculated as a mass per unit area (g/m2).

Lint-free performance was evaluated as follows.

Cellophane adhesive tape having a width of 10 mm was cut into about 30 mm and affixed to a surface of the medical gauze. After 60 seconds, the cellophane adhesive tape was removed from the medical gauze and affixed to slide glass. The affixed cellophane adhesive tape was given marks of a length of 10 mm and the number of lints within a range of 10 mm * 10 mm square was counted using an optical microscope of a magnification of 40. This measurement was conducted six times and an average thereof was employed as a numerical value showing the lint-free performance.

Water absorbing property was evaluated by the following method.

The medical gauze was cut into a size of 200 mm * 200 mm, a weight (W1) in a normal state (20°C * 65% RH) and a weight (W2) in a wet state were measured, the amount of water absorption (M1) was determined using the formula of M1 (%) = (W2 - W1)/W1 * 100, and an average thereof was calculated.

It is noted that the weight in the wet state (W2) is a weight determined after the medical gauze was dipped in water for 10 minutes, then taken out of the water, and left unattended for 10 minutes while being stretched on a wire mesh to remove excessive moisture from the medical gauze.

According to Example 1, a warp knitted fabric (without x-ray contrast yarn) was formed under the following conditions using a 28GG warp knitting machine.

Yarn used for forming the warp knitted fabric was Bemberg fiber 56 dtex/30f as cupra (available from Asahi Kasei Kabushiki Kaisha, Bemberg is a registered trademark).

Then, with the number of reeds of 2, the number of courses on the machine of 45, and the number of wales on the machine of 28, a marquisette construction was formed as shown in Fig. 2.

The warp knitted fabric formed in this way was subjected to scouring process at 70°C for 60 minutes in a solution containing 0.2% by weight of hydrogen peroxide water (a content from 30 to 30.5% by weight).

Then, the warp knitted fabric after the scouring was cut into 30 cm * 30 cm, and then the foregoing performance evaluation was conducted. Fig. 5 shows a photograph of the construction according to Example 1.

According to Example 2, a warp knitted fabric (without x-ray contrast yarn) was formed under the following conditions using a 28GG warp knitting machine.

Yarn used for forming the warp knitted fabric includes Bemberg fiber 56 dtex/30f available from Asahi Kasei Kabushiki Kaisha, and polyester fiber 56 dtex/24f (available from KB SEIREN LTD.). The Bemberg fiber was mixed to a ratio of 70% by weight.

Then, with the number of reeds of 2, the number of courses on the machine of 45, and the number of wales on the machine of 28, a marquisette construction was formed.

According to Example 2, a weight was set at 28 g/m², which is a lower limit of a preferable range.

The warp knitted fabric formed in this way was subjected to process in the same way as Example 1, and then performance evaluation was conducted.

According to Example 3, except for using viscose rayon fiber 56 dtex/24f instead of the Bemberg fiber used in Example 1, process was performed in completely the same way as in Example 1 and performance evaluation thereof was conducted.

According to Example 4, one polystyrene-based contrast yarn as a 0.85 mmϕ monofilament (with a content of barium sulfate of 50 percent by weight) was bonded by thermal compression using an ultrasonic welding machine to the prototype of the medical gauze of 30 cm * 30 cm formed in Example 1, and then performance evaluation was conducted.

According to Example 5, a warp knitted fabric (with x-ray contrast yarn) was formed under the following conditions using a 28GG warp knitting machine.

The warp knitted fabric was configured in a marquisette construction as shown in Fig. 7 using Bemberg fiber 56 dtex/30f available from Asahi Kasei Kabushiki Kaisha, using one polystyrene-based contrast yarn as a 0.65 mmϕ monofilament (with a content of barium sulfate of 50 percent by weight), and with the number of reeds of 3 (guide bars 5, 6, 7), the number of courses on the machine of 45, and the number of wales on the machine of 28. For reference, with the first guide bar 5 defined as GB1, the second guide bar 6 as GB2, and the third guide bar 7 as GB3, this marquisette construction is expressed in a numerical form as follows: GB1: 01-10, GB2: 33-00-11-00, and GB3: 22-00.

The warp knitted fabric formed in this way was subjected to process in the same way as Example 1 and then performance evaluation was conducted.

According to Example 6, except for using Bemberg fiber 84 dtex/45f as cupra (available from Asahi Kasei Kabushiki Kaisha) instead of the Bemberg fiber 56 dtex/30f used in Example 5, process was performed in completely the same way as in Example 5 and then performance evaluation was conducted. Fig. 4 shows the configuration according to Example 6 with interlaced x-ray contrast yarn. Fig. 6 shows a photograph of the construction according to Example 6.

According to Example 7, except for using yarn formed by twisting five Bemberg x-ray contrast yarns 300 dtex/60f together (sheath-core fiber with a content of barium sulfate of 60 percent by weight) at Z100t/m instead of the polystyrene-based x-ray contrast yarn used in Example 6, process was performed in the same way as in Example 6 to obtain performance evaluation result.

According to Comparative Example 1, plain woven gauze was formed using Bemberg fiber 167 dtex/70f available from Asahi Kasei Kabushiki Kaisha as warp yarn and weft yarn to a warp yarn density and a weft yarn density of 12/cm. Then, process was performed in the same way as in Example 1 and performance evaluation was conducted. Fig. 3 shows a photograph of the construction according to Comparative Example 1.

It is noted that the resultant gauze was determined to be incapable of retaining a shape as a woven fabric as at least one of the warp yarn and the weft yarn was moved easily only with touch by a hand to cause slippage phenomena frequently.

According to Comparative Example 2, by using 40-cotton yarn as warp yarn and weft yarn to a warp yarn density and a weft yarn density of 12/cm, plain woven gauze was formed as cotton gauze as an ordinary item.

This plain woven gauze was also subjected to degreasing process and bleaching process continuously under conditions same as scouring conditions for ordinary cotton gauze. Then, the gauze was cut into 30 cm * 30 cm and performance evaluation was conducted.

Table 1 shows result of the performance study conducted on each of Examples and each of Comparative Examples described above. In Table 1, PS shows polystyrene-based x-ray contrast yarn and BB shows Bemberg x-ray contrast yarn.

**[Table 1]**

| | | FABRIC CONSTRUCTION | REGENERATED CELLULOSE FIBER | OTHER FIBER | REGENERATED CELLULOSE WEIGHT PERCENT (%) | PRESENCE/ABSENCE OF X-RAY CONTRAST YARN | GAUZE PROPERTY | | | | |
|---|---|---|---|---|---|---|---|---|---|---|---|
| | | | | | | | GAUZE SIZE (mm) | GAUZE WEIGHT (g/m²) | LINT FREE PERFORMANCE | AMOUNT OF WATER ABSORPTION (%) | X-RAY CONTRAST PERFORMANCE |
| EXAMPLE | 1 | WARP KNITTING | CUPRA 56 | NO | 100 | NO | 300 * 300 | 30 | < 1 | 320 | - |
| | 2 | | CUPRA 56 | POLYE - STER 56 | 70 | NO | | 28 | < 1 | 230 | - |
| | 3 | | RAYON 56 | NO | 100 | NO | | 30 | 6 | 310 | - |
| | 4 | | CUPRA 56 | NO | 100 | YES (PS WELDED) | | 30 | < 1 | 320 | ○ |
| | 5 | | CUPRA 56 | NO | 100 | YES (PS) | | 30 | < 1 | 320 | ⊚ |
| | 6 | | CUPRA 84 | NO | 100 | YES (PS) | | 90 | < 1 | 280 | ⊚ |
| | 7 | | CUPRA 84 | NO | 100 | YES (BB) | | 30 | < 1 | 280 | ○ |
| COMPARA -TIVE EXAMPLE | 1 | PLAIN WEAVING | CUPRA 167 | NO | 100 | NO | 300 * 300 | 40 | < 1 | 360 | - |
| | 2 | | NATURAL FIBER (COTTON) | | - | YES (PS) | | 36 | 23 | 340 | ⊚ |

As shown in Table 1 and each photograph, according to each of Examples, the construction was retained successively with favorable lint-free performance and without the occurrence of slippage, and the like. By contrast, Comparative Example 1 corresponding to the weaving using cupra failed to retain the construction due to the occurrence of slippage while favorable lint-free performance was achieved. Comparative Example 2 corresponding to the plain weaving using natural fiber resulted in considerably bad lint-free performance.

### [Strength test]

Described next is a strength test conducted using the warp knitted gauze (medical gauze) according to Examples.

### (1) Specimen

Specimens used include a specimen prepared by interlacing x-ray contrast yarn in a warp knitted fabric made of cupra 84 shown in Fig. 11(a), a specimen prepared by interlacing four PET fibers in an area around x-ray contrast yarn shown in Fig. 11(b), and a specimen prepared by interlacing eight PET fibers in an area around x-ray contrast yarn shown in Fig. 11(c) . It is noted that cupra 84 is Bemberg fiber (long fiber) 84 dtex/45f. The x-ray contrast yarn is a 0.65 mmϕ monofilament.

### (2) Scouring condition

Scouring process was performed on the specimens using hydrogen peroxide water of 0.2% as a processing solution at a processing temperature from 65 to 70°C and for a processing duration of 60 minutes.

### (3) Test result

Fig. 12 shows test result. The test specimens were sterilized with EOG having a width of 30 mm. Tensile strength in a CD direction was measured in a part including the x-ray contrast yarn (see Fig. 12(b)).

A specimen wet with water (wet gauze) was reduced in strength in long fiber intertwined with the x-ray contrast yarn and was broken easily at this part (see Fig. 12 (c)). This is considered to be caused for the reason that, as a result of a large difference in diameter between the long fiber and the x-ray contrast yarn, the long fiber was in a state of causing yarn breakage easily under heavy load and the long fiber became prone to cutting by being wet in water during implementation of the scouring process and implementation of the strength test.

As shown in the photograph of Fig. 12 (c), if break occurs in an area adjacent to the x-ray contrast yarn, concern about a risk of dropping of the x-ray contrast yarn from the warp knitted fabric is caused. On the other hand, in the configuration where the PET fiber is interlaced in an area adjacent to the x-ray contrast yarn, dropping of the x-ray contrast yarn from the warp knitted fabric does not occur.

As shown in the table in Fig. 12 (a), lower break strength of the specimen with eight PET fibers than those in the other specimens is considered to be caused for the reason that, as a result of an increased number of PET fibers not to be stretched, the long fiber at a boundary area is subjected to load due to failing to absorb its stretch sufficiently.

Thus, in order to reinforce an area around the x-ray contrast yarn and prevent dropping of the x-ray contrast yarn, PET fiber higher in fiber strength than other long fiber is necessary and an appropriate number of PET fibers according to Examples is considered to be four.

### [Surface resistance check test]

Described next is a surface resistance check test conducted using the warp knitted gauze (medical gauze) according to Examples and the plain woven cotton gauze according to Comparative Examples.

### (1) Specimen

Specimens used include a warp knitted gauze low-weight item (weight of 56 g/m²) , a warp knitted gauze large-weight item (weight of 84 g/m²) , and plain woven cotton gauze (weight of 38 g/m²) . It is noted that the warp knitted gauze is gauze with a warp knitted fabric made of cupra 84.

### (2) Test method

Petri dishes having glass petri dish bottom surfaces with affixed PP plates (a plate with a smooth surface and a plate with an uneven surface) were placed over the specimens (gauze), and moved at a constant speed by a tensile testing machine. Tensile test strength (testing force) was determined to be a resistance value at a gauze surface. The petri dishes with the affixed PP plates have a weight of 200 g, were installed in an area of 60 cm², and were pulled at a speed of 30 cm/min.

### (3) Test result

Fig. 13 shows test result obtained by using the PP plate with the smooth surface. Fig. 14 shows test result obtained by using the PP plate with the uneven surface. It is noted that, in each of these drawings, a is a graph of the plain woven cotton gauze, b is a graph of the warp knitted gauze large-weight item, and c is a graph of the warp knitted gauze low-weight item.

Accordingly, in the warp knitted gauze of either case, a surface resistance value is lower than that of the plain woven cotton gauze, showing that the surface of the warp knitted gauze is more slippery than the surface of the plain woven cotton gauze. In a scene of using a port in an endoscopic operation, for example, gauze is preferably easy to slip inside a cylindrical port. In this regard, the warp knitted gauze can be loaded and unloaded more easily than the plain woven cotton gauze, so that the warp knitted gauze according to Examples is considered to be more superior to the plain woven cotton gauze according to Comparative Examples.

### [Fabric detachability check test]

Described next is a fabric detachability check test conducted using the warp knitted gauze (medical gauze) according to Examples and the plain woven cotton gauze according to Comparative Examples.

### (1) Specimen

Specimens used include a warp knitted gauze low-weight item (weight of 56 g/m²) and plain woven cotton gauze (weight of 36 g/m²). The warp knitted gauze is gauze with a warp knitted fabric made of cupra 84.

### (2) Test method

A plate used is a test metallic plate used in the clinging measurement according to JIS L 1094. This metallic plate is a stainless steel plate having a size of about 100 mm * 450 mm and a thickness of about 1.3 mm, and with a 70-degree bend at a position of about 150 mm from one end thereof.

Two pieces (about 75 mm * 230 mm) were taken from each specimen, the upper and lower ends of one of the specimen pieces were fixed with adhesive tape to the metallic plate, the other specimen piece was superimposed on the fixed specimen piece, and then the upper ends of the two specimen pieces were attached together to the metallic plate with a clamp.

Two specimen pieces attached in this way to the metallic plate were rubbed three times in the palms of hands at a speed of once in one second, then the metallic plate was erected to an upright position, and the angle of the metallic plate was read when the two specimen pieces were detached from each other by tilting the metallic plate gradually.

### (3) Test result

Fig. 15 shows test result of fabric detachability about the two warp knitted gauze pieces. Fig. 16 shows test result of fabric detachability about the two plain woven cotton gauze pieces . Accordingly, in the case of the two warp knitted gauze pieces, by tilting the metallic plate slightly (an angle of tilt from three to four degrees), the fabrics were detached from each other. On the other hand, in the case of the two plain woven cotton gauze pieces, even by tilting the metallic plate largely (even tilted to an angle of 90 degrees), the fabrics remained attached without being detached from each other.

The foregoing shows that warp knitted gauze has favorable fabric detachability compared to plain woven cotton gauze and thus achieves more excellent ease of handling during actual use. Thus, in a scene of using gauze pieces superimposed on each other in a bundle, for example, the warp knitted gauze is more detachable than the plain woven cotton gauze. As a result, the warp knitted gauze according to Examples is considered to be more superior (in terms of improvement of workability, and the like) to the plain woven cotton gauze according to Comparative Examples.

### [Performance check test]

Described next is a performance check test conducted using the warp knitted gauze (medical gauze) according to Examples and the plain woven cotton gauze according to Comparative Examples.

### (1) Specimen

Specimens used include a warp knitted gauze low-weight item, a warp knitted gauze large-weight item, and plain woven cotton gauze. It is noted that the warp knitted gauze is gauze with a warp knitted fabric made of cupra 84.

### (2) Test method

### A. Basic performance check (weight, air permeability, open area ratio)

For an air permeability test, an airflow quantity (cc/cm²/sec) was measured under pressure loss of 12.5 mm H₂O according to the JIS L 1096A method (fragile method). As a textile in a sparse construction provides a large airflow quantity, four pieces were stacked for the measurement. In addition, for an open area ratio check test, image analysis was performed using a microscope available from KEYENCE

### CORPORATION.

### B. Mechanical characteristic check

Kawabata evaluation system (KES) tensile characteristic, KES bending characteristic, and bending resistance (JIS 1096) were measured. It is noted that a WET state was created by dipping gauze into water, then measuring a weight after dewatering for 30 seconds using a dryer of a twin-tub washing machine, and making adjustment to achieve a water content of about 80 wt%.

### (3) Test result

Fig. 17 shows test result. Regarding the air permeability and the open area ratio, Example 2 achieves higher air permeability and a higher open area ratio than Comparative Example 1 despite a larger weight than Comparative Example 1. Regarding linearity LT (weft direction) (linearity LT: a lower value shows increased ease of stretching at an initial stage of pulling), a value tends to become smaller in the weft direction according to Examples than a corresponding value according to Comparative Examples. From this view, the warp knitted gauze is found to be stretched more easily in the weft direction than the plain woven cotton gauze at an initial stage of pulling. Regarding workload WT (workload WT: a larger value means higher stretching property), a value in each of the warp direction and the weft direction according to Examples tends to become larger than a corresponding value according to Comparative Examples. From this view, the warp knitted gauze is found to be stretched more easily than the plain woven cotton gauze in the warp direction and the weft direction. Regarding bending rigidity B (bending rigidity B: a larger value means increased hardness of bending) , a value is smaller in the weft direction according to each of Examples 2 and 4 than a corresponding value according to each of Comparative Examples 1 and 2. From this view, the warp knitted gauze is found to be bent more easily in the weft direction than the plain woven cotton gauze. Regarding the D method (heart-loop method), the weft direction according to Examples tends to provide a larger value than a corresponding value according to Comparative Examples. From this view, the warp knitted gauze is found to be bent more easily in the weft direction than the plain woven cotton gauze.

### INDUSTRIAL APPLICABILITY

The present invention is applied as medical gauze capable of absorbing blood or body fluids, for example.

### REFERENCE SIGNS LIST

1 Medical gauze
2 Loop
3 Warp knitted fabric
4 X-ray contrast yarn
21 to 24, 31 to 38 Chemical fiber

## Claims

1. Medical gauze (1) comprising:
a warp knitted fabric (3) having a warp direction and a weft direction and composed of loops (2) continued in the warp direction; and
x-ray contrast yarn (4) interlaced in the warp knitted fabric, wherein the warp knitted fabric is made of 100% long fiber containing 70% by weight or more of regenerated cellulose-based long fiber;
**characterized in that**:
at least one chemical fiber (21 to 24, 31 to 38) is intertwined with the x-ray contrast yarn (4) in the warp direction; and
similar chemical fiber is arranged in the warp direction in the vicinity of the at least one chemical fiber.

2. The medical gauze according to claim 1, wherein
the warp knitted fabric is configured in a marquisette construction.

3. The medical gauze according to claim 1 or 2, wherein
the regenerated cellulose-based long fiber is cuprammonium rayon.

4. The medical gauze according to any one of claims 1 to 3, wherein
the medical gauze (1) has a width of equal to or greater than 5 mm and equal to or less than 600 mm, and a weight of equal to or greater than 28 g and equal to or less than 290 g per square meter.

5. The medical gauze according to any one of the preceding claims, wherein
the total number of the chemical fibers is between four and eight.

6. The medical gauze according to any one of the preceding claims, wherein
the chemical fiber is polyethylene terephthalate fiber.

## Patentansprüche

1. Medizinische Gaze (1) umfassend:
Ein Kettengewirke (3) mit einer Kettrichtung und einer Schussrichtung und bestehend aus Maschen (2), die in Kettrichtung fortgeführt werden; und
Röntgenkontrastfaden (4), der mit dem Kettengewirke verflochten ist, wobei das Kettengewirke aus 100 % Langfaser mit 70 Vol.-% oder mehr aus wiedergewonnenen zellulosebasierten Langfasern hergestellt ist; **dadurch gekennzeichnet dass**:
Mindestens eine chemische Faser (21 bis 24, 31 bis 38) mit dem Röntgenkontrastfaden (4) in Kettrichtung verflochten ist; und
eine ähnliche chemische Faser in Kettrichtung neben der mindestens einen chemischen Faser angeordnet ist.

2. Medizinische Gaze nach Anspruch 1, wobei
das Kettengewirke in einer Marquisette-Ausführung konfiguriert ist.

3. Medizinische Gaze nach Anspruch 1 oder 2,
wobei die wiedergewonnene zellulosebasierte Langfaser Kupfer-Ammonium-Rayon ist.

4. Medizinische Gaze nach einem der Ansprüche 1 bis 3, wobei
die medizinische Gaze (1) eine Breite gleich oder größer als 5 mm und gleich oder geringer als 600 mm und ein Gewicht von gleich oder höher als 28 g und gleich oder geringer als 290 g pro Quadratmeter hat.

5. Medizinische Gaze nach einem der der vorhergehenden Ansprüche, wobei
die Gesamtzahl der chemischen Fasern zwischen vier und acht liegt.

6. Medizinische Gaze nach einem der vorhergehenden Ansprüche, wobei
die chemische Faser eine Polyethylenterephthalat-Faser ist.

## Revendications

1. Gaze médicale (1) comprenant :
l'invention concerne un tissu tricoté chaîne (3) présentant un sens chaîne et un sens trame et composé de boucles (2) poursuivies dans le sens chaîne ; et
fil de contraste aux rayons x (4) entrelacé dans le tissu tricoté chaîne, dans laquelle le tissu tricoté chaîne est constitué de 100 % de fibres longues contenant 70 % en poids ou plus de fibres longues à base de cellulose régénérée ; **caractérisé en ce que** :
au moins une fibre chimique (21 à 24, 31 à 38) est entrelacée avec le fil de contraste aux rayons x (4) dans le sens chaîne ; et
une fibre chimique similaire est disposée dans le sens chaîne à proximité de ladite au moins une fibre chimique.

2. Gaze médicale selon la revendication 1, dans laquelle
le tissu tricoté chaîne est conçu en construction de marquisette.

3. Gaze médicale selon la revendication 1 ou la revendication 2,
dans laquelle la fibre longue à base de cellulose régénérée est la rayonne cuprammonium.

4. Gaze médicale selon l'une quelconque des revendications 1 à 3, dans laquelle
la gaze médicale (1) présente une largeur égale ou supérieure à 5 mm et égale ou inférieure à 600 mm, et un poids égal ou supérieur à 28 g et égal ou inférieur à 290 g par mètre carré.

5. Gaze médicale selon l'une quelconque des revendications précédentes, dans laquelle :
le nombre total de fibres chimiques est compris entre quatre et huit.

6. Gaze médicale selon l'une quelconque des revendications précédentes, dans laquelle :
la fibre chimique est une fibre de polyéthylène téréphtalate.
